# EUROPÄISCHE PATENTSCHRIFT

(11) **EP 2 399 648 B1**
(45) Veröffentlichungstag und Bekanntmachung des Hinweises auf die Patenterteilung: **04.04.2018**
(21) Anmeldenummer: 11170764.2
(22) Anmeldetag: 21.06.2011
(51) Int. Cl.: A61Q 19/00, A61K 8/67, A61K 8/97, A61K 9/06, A61K 31/714, A61K 9/00, A61K 36/29, A61K 36/886, A61P 17/06, A61P 17/04

(54) **Kosmetische Zusammensetzung und deren Verwendung**
Cosmetic composition and use of same
Composition cosmétique et son utilisation

(30) Priorität: 23.06.2010 DE 102010030443
(43) Veröffentlichungstag der Anmeldung: 28.12.2011
(73) Patentinhaber: LR Health & Beauty Systems GmbH, 59227 Ahlen (DE)
(72) Erfinder: Dr. Lierhammer, Raimund, 59269 Beckum (DE)
(74) Vertreter: Maikowski & Ninnemann Patentanwälte Partnerschaft mbB

(56) Entgegenhaltungen:
- DE-U1- 20 109 044
- FR-A1- 2 673 840
- GB-A- 2 453 157
- US-A- 4 885 157
- US-A1- 2007 148 226
- US-A1- 2009 169 489
- US-A1- 2010 150 895
- US-B1- 6 300 324
- US-B1- 6 733 797
- DATABASE CA [Online] CHEMICAL ABSTRACTS SERVICE, COLUMBUS, OHIO, US; 7. September 2006 (2006-09-07), "Pharmaceutical compositions containing Chinese medicines for antitussive and antiasthmatic effects", XP002661632, Database accession no. 145:235781
- DATABASE GNPD [Online] Mintel; Juli 2002 (2002-07), "Cellular Hydrating Serum", XP002672725, Database accession no. 159779
- Anonym: "Aloe vera (Aloe barbadensis)", , 28. Februar 2009 (2009-02-28), XP002672737, Gefunden im Internet: URL:http://web.archive.org/web/20090228202 259/http://www.herbwisdom.com/herb-aloe-ve ra.html [gefunden am 2012-04-02]
- H N Saada ET AL: "Effectiveness of Aloe vera on the antioxidant status of different tissues in irradiated rats", Die Pharmazie, 1 December 2003 (2003-12-01), page 929, XP055189576, Germany Retrieved from the Internet: URL:http://www.ncbi.nlm.nih.gov/pubmed/147 03976
- STÜCKER M ET AL: "Topical vitamin B12--a new therapeutic approach in atopic dermatitis-evaluation of efficacy and tolerability in a randomized placebo-controlled multicentre clinical trial", BRITISH JOURNAL OF DERMATOLOGY, OXFORD : WILEY-BLACKWELL, UK, vol. 150, no. 5, 1 May 2004 (2004-05-01), pages 977-983, XP007910269, ISSN: 0007-0963, DOI: 10.1111/J.1365-2133.2004.05866.X
- DEVARAJ SRIDEVI ET AL: "Aloe supplements enhance bioavailability of vitamin C and B12 in older adults", THE FASEB JOURNAL, FEDERATION OF AMERICAN SOCIETIES FOR EXPERIMENTAL BIOLOGY, US, vol. 21, no. 5, 1 April 2007 (2007-04-01), page A111, XP002532761, ISSN: 0892-6638
- SMIROLDO ET AL: "Treatment review of chronic psoriasis", AUSTRALIAN JOURNAL OF MEDICAL HERBALISM, NATIONAL HERBALISIS ASSOCIATION OF AUSTRALIA, KINGSGROVE, AU, vol. 18, no. 3, 22 September 2006 (2006-09-22), pages 103-106, XP009193482, ISSN: 1033-8330
- "Psoriasis Natural Treat", , 19 March 2006 (2006-03-19), Retrieved from the Internet: URL:https://web.archive.org/web/2006031913 5208/http://www.healthy-skin-guide.com/pso riasis-natural-treat.html [retrieved on 2017-09-19]
- "Nachtkerzenöl- Wirkstoffe", , 27 May 2010 (2010-05-27), Retrieved from the Internet: URL:https://web.archive.org/web/2010052721 4205/http://www.nachtkerzenoel.net:80/wirk stoffe.html [retrieved on 2017-09-19]
- "Nachtkerzenöl- Anwendung", , 10 June 2010 (2010-06-10), Retrieved from the Internet: URL:https://web.archive.org/web/2010061009 5719/http://www.nachtkerzenoel.net:80/anwe ndung.html [retrieved on 2017-09-19]

## Beschreibung

Die vorliegende Erfindung betrifft eine kosmetische Zusammensetzung nach Anspruch 1 und ein Verfahren zu deren Herstellung nach Anspruch 8.

Die menschliche Haut stellt das größte Organ des Menschen dar und funktioniert als Schutzschicht gegen chemische und physikalische Einflüsse. Die primäre Funktion der mehrschichtig aufgebauten Haut besteht in der Wirkung als mechanische und chemische Schutzbarriere sowie als Barriere gegen Wasserverlust.

Komplexe Wirkmechanismen in der Haut tragen zum Erhalt des Hydrationsstatus der äußeren Hautschicht bei. Die Einwirkung von extremen äußeren Bedingungen, wie z.B. Kälte, trockenes Winterwetter, Chemikalien oder Lösungsmitteln kann jedoch zur Austrocknung der Haut führen. Neben den externen Faktoren, die zur Austrocknung der Haut führen, sind einige Krankheiten bekannt, die zu abnormen Hautbildern führen, wie z.B. Dermatitis oder Psoriasis.

Diverse Feuchtigkeitsmittel in Form von Cremes oder Lotionen werden zur Verhinderung der Austrocknung der Haut verwendet. Als Feuchtigkeit spendende Verbindungen kommen in diesem Feuchtigkeitsmittel insbesondere Verbindungen wie Glycerol, Harnstoff, Polyethylenglykole, Lanolin, Milchsäure oder Ceramide zum Einsatz, die üblicherweise mit einer Vielzahl von weiteren Inhaltsstoffen zur Erzielung einer guten Effizienz und Stabilität versetzt werden. Die hohe Anzahl an Inhaltsstoffen wirkt sich jedoch häufig nachteilig auf den Zustand der Haut aus und kann zu allergischen Reaktionen führen. Zudem basieren die üblicherweise verwendeten Feuchtigkeitsmittel auf chemisch synthetisierten Komponenten.

Aus diesem Grund kommt der Verwendung von in der Natur auftretenden Verbindungen, insbesondere als Bestandteil von pflanzlichen Extrakten, zur Behandlung von irritierter und gestörter Haut eine zunehmend größere Bedeutung zu, da diese häufig gut verträglich sind und nur ein reduziertes allergenes Potential aufweisen.

Arzneimittel auf pflanzlicher Basis (Phytopharmaka) spielen traditionellerweise bei der Behandlung von Wunden, Hautinfektionen sowie Ekzemen eine große Rolle und weisen ein breites Wirkspektrum auf. Phytopharmaka weisen eine Vielzahl von Inhaltsstoffen auf, die sowohl zur Behandlung der Symptome als auch deren Ursachen wirksam sein können.

So ist z.B. die entzündungshemmende Wirkung von Extrakten aus Kamillenblüten, Spitzwegerichkraut, Berberitze oder Eichenrinde bekannt. Ein typischer Vertreter der Familie der Berberitzengewächse bzw. Mahonien ist *Mahonia aquifolium,* der als immergrüner, 1 bis 2 Meter hoher Strauch u.a. in Nordamerika anzutreffen ist. Dort wurde die Pflanze in der traditionellen Medizin nordamerikanischer Indianer bei Hautkrankheiten und Magen-Darm-Störungen eingesetzt. In der Homöopathie findet Mahonia unter anderem bei der Behandlung von Hautkrankheiten, die mit Juckreiz, Bläschen und Pusteln einhergehen, Anwendung. Im Falle der Mahonia-Pflanze können derzeit einzelne pharmakologische Effekte definierten Inhaltsstoffen zugeordnet werden. So ist insbesondere bekannt, dass die Alkaloide Berberin, Berbamin und Oxyacanthin Entzündungen der Haut vermindern und das Zellwachstum hemmen. In der US 2007/0148226 A1 wird z.B. die Verwendung von Mahonia Extrakt zur Behandlung von Hautirritationen beschrieben.

Vitamin B12 ist ein für Tiere und Menschen essentielles Vitamin, das als Coenzym an den enzymatischen Reaktionen der Methionin-Synthase und der Methylmalonyl-COa-Mutase beteiligt ist. Vitamin B12 kann vom tierischen und menschlichen Organismus nicht sensibilisiert werden und muss daher extern zugeführt werden, wobei insbesondere Fleisch eine besonders reichhaltige Quelle an Vitamin B12 darstellt. Bei Mangel an Vitamin B12 kann es zu einer perniziösen Anämie, einer Erkrankung des Blutbildes, und zur Myelose kommen.

Eine Wirkung des Vitamins B12 in der Dermatologie zur Behandlung von atopischen Ekzemen (Neurodermitis) und Schuppenflechte (Psoriasis) wurde in der Vergangenheit vorgeschlagen (Stücker, M. et al, Topical Vitamin B12 - a new therapeutic approach in atopic dermatitis - evaluation of efficiacy and tolerability in a randomized placebo-controlled multicentre clinical trial, The British Journal of Dermatology, 2004, Bd. 150, S. 977-983). Umfangreiche Studien zur Wirksamkeit des Vitamin B12 bzw. einer unter dem Namen Regividerm bekannten Vitamin B12-haltigen Creme liegen jedoch nicht vor. Es wird vermutet, dass das Vitamin B12 aufgrund seiner spezifischen Struktur in der Lage ist, Stickstoffmonoxid zu binden, welches bei symptomatischen Hautveränderungen in erhöhter Konzentration auftritt und zellschädigend wirkt.

Obwohl einige Indikationen im Hinblick auf die Wirksamkeit von pflanzlichen Extrakten wie Mahonia-Extrakt und Vitamin B12 zur Behandlung von Hautkrankheiten, insbesondere von Neurodermitis, existieren, ist deren Wirksamkeit aufgrund von mangelnden, längerfristigen Studien bisher nicht eindeutig belegt.

Zudem sind die beschriebenen Komponenten für die betroffene Personengruppe nicht in einer Form, wie z.B. lagerfähige Cremes oder Lotionen, erhältlich, die eine effektive und nachhaltige Verwendung ermöglichen.

Es ist somit eine Aufgabe der vorliegenden Erfindung eine kosmetische Zusammensetzung bereitzustellen, die sowohl zur Behandlung von Hautirritationen geeignet ist als auch eine hohe Stabilität und Lagerfähigkeit aufweist.

Diese Aufgabe wird mit einer kosmetischen Zusammensetzung mit den Merkmalen der Anspruchs 1 gelöst.

Demnach umfasst die erfindungsgemäße kosmetische Zusammensetzung,
- 0,01 bis 2 Gew% Cyanocobalamin,
- 0,01 bis 5 Gew% von mindestens einem ethanolisch-wässrigen Extrakt standardisiert auf die Leitsubstanz Berberin aus der Pflanze *Mahonia aquifolium* , wobei der Anteil an Berberin im Extrakt mehr als 0,5 mg/ml beträgt,
- 1 bis 50 Gew% eines Aloe vera Gels, sowie
- Nachtkerzenöl, Acrylate / C10-C30 Alkylacrylatcrosspolymer und Glyceride.

Die erfindungsgemäße Zusammensetzung ist besonders zur äußeren Behandlung und Prophylaxe von irritierter und trockener Haut sowie von Hauterkrankungen, insbesondere Neurodermitis, geeignet. Des Weiteren dient die erfindungsgemäße Zusammensetzung der Zufuhr von Feuchtigkeit zu trockener Haut und weist ein angenehmes Gefühl auf der Haut für den Konsumenten auf.

Das in der erfindungsgemäßen Zusammensetzung verwendete Cyanocobalamin hat sich dabei aufgrund seiner Stabilität und Verfügbarkeit als besonders geeignet erwiesen.

Das mindestens eine Cyanocobalamin kann in einer Menge von bevorzugt 0,05 bis 1,5 Gew %, insbesondere bevorzugt 0,1 bis 1 Gew%, in der Zusammensetzung enthalten sein.

Erfindungsgemäß besonders geeignet für den Einsatz in der erfindungsgemäßen Zusammensetzung ist ein Extrakt aus *M. aquifolium* (auch umgangssprachlich als *Berberis aquifolium* oder *B. aquifolium* bezeichnet). Der Extrakt liegt erfindungsgemäß als ethanolisch-wässriger Extrakt vor. Der verwendete Extrakt ist auf den wichtigsten Inhaltsstoff bzw. die Leitsubstanz Berberin standardisiert. So beträgt der Anteil an Berberin im Extrakt erfindungsgemäß mehr als 0,5 mg/ml, insbesondere bevorzugt mehr als 1 mg/ml, ganz besonders bevorzugt mehr als 1,4 mg/ml.

Das Mahonia-Extrakt kann bevorzugt in einer Menge zwischen 0,05 bis 2 Gew%, insbesondere bevorzugt 0,1 bis 1 Gew%, in der Zusammensetzung zum Einsatz kommen.

Erfindungsgemäß ist der Zusammensetzung mindestens ein weiteres pflanzliches Extrakt als entzündungshemmendes und/oder feuchtigkeitsspendendes Mittel, auf der Basis von Aloe vera, zugesetzt. Aloe vera wird traditionell zur äußeren Behandlung von Abszessen, Akne, Neurodermitis, Ekzemen oder schlecht heilenden Wunden verwendet. Des Weiteren erhöht Aloe vera, insbesondere in Form eines Aloe vera Gels, die Bioverfügbarkeit von Vitamin B12 sowie die antioxidative Absorptionskapazität von Plasma behandelt mit Aloe vera Gel, Vitamin C und Vitamin B12.

Aloe vera in Form eines Gels, wird vorteilhafterweise in einer Menge zwischen zwischen 5 und 30 Gew%, insbesondere bevorzugt zwischen 10 und 25 Gew% Aloe vera in der Zusammensetzung verwendet.

Weitere pflanzliche Extrakte, die der Zusammensetzung zugegeben werden können, sind u.a. Extrakte aus Ballonrebenkraut, Bittersüßstengel, Eichenrinde, Kamillenblüten, Spitzwegerichkraut, Taubnesselblüten, Arnikablüten, Walnussblätter, Wassernabelkraut und/oder Pfingstrosenwurzel.

Erfindungsgemäß umfasst die kosmetische Zusammensetzung Nachtkerzenöl .In einer weiteren Ausführungsform umfasst die kosmetische Zusammensetzung weitere feuchtigkeitsspendende Verbindungen, insbesondere auf pflanzlicher Basis. Typische Feuchtigkeitsspender sind Avocadin (Persea Gratissima Avocado Oil Unsaponifiables), Glycine Soja (Soybean) Sterole, Red Alfa Lingonberry Seed Oil (Vaccinium Vitis-idea Seed Oil), Kamillenöl, Weizenkeimöl, Sojaöl, Jojobaöl, Palmenöl oder Avocadoöl.

Besonders vorteilhaft ist die Verwendung von Nachtkerzenöl, das sich als besonders verträglich für Menschen mit Hautproblemen, insbesondere Neurodermitiker erwiesen hat. Nachtkerzenöl weist einen hohen Anteil an γ-Linolensäure auf, die eine gute Wirksamkeit bei der Behandlung von Schuppungen, Rötungen und Juckreiz aufweist.

Als weitere die Feuchtigkeit der Haut verbessernde Substanzen können Glycerin, Sodium PCA, Hyaluronsäure oder Mischungen bekannt als Aqua Cacteen enthaltend Opuntia Ficus-Indica Stamm-Extrakt; Aquafill enthaltend Glycosphingolipide, Hordeum Vulgare Extrakt und Sodium Hyaluronate; Aqualance enthaltend Erythritol und Homarine HCl; Aquarich enthaltend Avena Strigosa (Samen) Extrakt; Cell Active Hydro enthaltend Pyrus Malus, Pectin und Chlorella Vulgaris/Lupinus Albus Protein Ferment; Cupuacu Butter enthaltend Theobroma Grandiflorum Samenbutter; DayMoist enthaltend Beta Vulgaris Wurzel-Extrakt; Dragoderm enthaltend Triticum Vulgare (Weizen) Gluten; Fucogel enthaltend Biosaccharide Gum-1, Hydractin enthaltend Disodium Adenosine Triphosphate (1-5%); 0,1-1% Algin; Papaya enthaltend Carica Papaya Extrakt; Hydroxyethylurea; Lipomoist enthaltend Carrageenans (Chrondrus Crispus) und Glukose; Pentavitin enthaltend Saccharide Isomerat; Squalane; Polyplant PS enthaltend Olea Europaea und Phytosterole, Trimoist enthaltend Natrium Stearoyl Lactylate, Pflanzenöl, Glycine Soja (Sojybohnen) Sterole und Natrium Carboxymethyl Betaglucan; verwendet werden.

Darüber hinaus kann die Zusammensetzung mindestens einen Emulgator, mindestens ein Verdickungsmittel und/oder mindestens ein Konservierungsmittel enthalten.

Typische Emulgatoren sind Ester oder Mischester von gesättigten und ungesättigten Fettsäuren mit 6 bis 22 Kohlenstoffatomen, insbesondere Sorbitan Stearate, Glyceryl Stearate, Glyceryl Isostearate, Glyceryl Oleate, Sorbitan Oleate, Methyl Glucose Sesquistearate, Polyglyceryl Dioleate, Polyglyceryldistearate, Methyl Glucose Dioleate, Triglyceryl Diisostearate, Polyglycerly-3-diisostearate und/oder Polyglycerindimerat; und/oder Polysiloxan-Polyalkyl-Polyether-Copolymere, insbesondere Cetyl Dimethicone Copolyol.

Typische Verdickungsmittel und/oder Stabilisatoren basieren auf natürlichen Gummis und/oder synthetischen Polymeren, insbesondere Polysaccharide wie Xanthan gum, Agar-Agar, Guar-Guar, Alginate, Carboxymethylcellulose oder Hydroxyethylcellulose; Polyacrylate wie Acrylate/C10-C30 Alkyacrylatcrosspolymer; Polyacrylamide, Polyvinylalkohole u.a.

Das erfindungsgemäß verwendete Acrylate/C10-C30 Alkyacrylatcrosspolymer ist ein wasserlöslicher Gelbildner mit Co-O/W-Emulgator Eigenschaften. Es wirkt zudem stabilisierend auf das System zum Verdicken. Xanthan Gum dient insbesondere der Viskositätseinstellung und erzeugt weiche Texturen. Zur Einarbeitung in die Zusammensetzung ist der Zusatz von Glycerin und/oder Allantoin vor Erwärmen mit Wasser erforderlich. Auch sollte Xanthan Gum in Ölen vordispergiert werden.

Typische Konservierungsmittel sind Methylparaben, Propylparaben, Ethylhexylglycerin, Phenoxyethanol, Pentandiol und/oder Sorbinsäure. Des Weiteren können auch Zitronensäure, Kaliumsorbat und/oder Natriumbenzoat als Konservierungsmittel, insbesondere zur Vorkonservierung von Aloe vera, verwendet werden.

Weitere Konsistenzgeber und oberflächenaktive Mittel, die Bestandteil der Zusammensetzung sein können, sind u.a. Polyglykole wie Polyethylenglykol; Laurate, wie Natriumlaurat oder Kaliumlaurat; Alkohole, wie Cetearylalkohol, Cetylalkohol. Laurylalkohol, Myristylalkohol, Behenylalkohol und Lanolinalkohol; längerkettige (>C₆) gesättigte oder ungesättigte Fettsäuren, wie Palmitinsäure und Stearinsäure; sowie Lecithin.

Als erfindungsgemäße Konsistenzgeber werden Glyceride, insbesondere auf der Basis von natürlichen Fettsäuren verwendet. So können z.B. durch Veresterung von Palmenkernöl oder Palmenöl Palmenkernglyceride oder Palmenölglyceride verwendet werden. Durch Zugabe von geeigneten Konsistenzgebern wird die Verteilbarkeit der Zusammensetzung auf der Haut und deren Hitzestabilität erhöht.

Als Lösungsmittel können typischerweise Wasser und/oder Alkohole, insbesondere niedere Alkohole wie Ethanol, Propanol, iso-Propanol oder Butanol verwendet werden.

Auch ist es möglich, der Zusammensetzung weitere Additive, insbesondere mindestens ein Antioxidant, UV-absorbierende Verbindung, zuzusetzen.

Typischerweise umfasst die kosmetische Zusammensetzung mindestens eine der folgenden Verbindungen:

| **Inhaltsstoff** | **Inhaltsstoff gemäß INCI-Nomenklatur** | **Menge [Gew%]** |
|---|---|---|
| Wasser | Aqua (Water) | 25-50 |
| Nachtkerzenöl | Oenothera Biennis (Evening Primrose) Oil | 25-50 |
| Aloe barbadensis Gel | Aloe barbadensis Gel | 10-25 |
| Glycerin | Glycerin | 1-5 |
| Sorbitanstearate | Sorbitan Stearate | 1-5 |
| Glycerylstearate | Glyceryl Stearate | 1-5 |
| Cetearylalkohol | Cetearyl Alcohol | 1-5 |
| Alkohol denat. (Ethanol) | Alkohol denat. | 1-5 |
| Methylglucosesesquistearate | Methyl Glucose Sesquistearate | 1-5 |
| Hydrierte Palmenkernglyceride | Hydrogenated Palm Kernel Glycerides | 1-5 |
| *M. aquifolium* Extrakt | Berberis Aquifolium Extract | 0,1-1 |
| Behenylalkohol | Behenyl Alcohol | 0,1-1 |
| Palmitinsäure | Palmitic Acid | 0,1-1 |
| Lecithin | Lecithin | 0,1-1 |
| Laurylalkohol | Lauryl Alcohol | 0,1-1 |
| Hydrierte Palmglyceride | Hydrogenated Palm Glycerides | 0,1-1 |
| Acrylate/C 1 0-C30 Alkylacrylatcrosspolymer | Acrylate/C10-C30 Alkylacrylatcrosspolymer | 0,1-1 |
| Stearinsäure | Stearic Acid | 0,1-1 |
| Myristylalkohol | Myristyl Alcohol | 0,1-1 |
| Cetylalkohol | Cetyl Alcohol | 0,1-1 |
| Xanthan Gum | Xanthan Gum | 0,1-1 |
| Cyanocobalamin | Cyanocobalamin | 0,1-1 |
| Phenoxyethanol | Phenoxyethanol | 0,1-1 |
| Ethylhexylglycerin | Ethylhexylglycerin | 0,1-1 |
| Zitronensäure | Citric Acid | < 0,1 |
| Kaliumsorbat | Potassium Sorbate | < 0,1 |
| Natriumbenzoat | Sodium Benzoate | < 0,1 |
| Natriumhydroxid | Sodium Hydroxide | < 0,1 |

Glycerylstearate, Behenylalkohol, Palmitinsäure, Stearinsäure, Lecithin, Laurylakohol, Myristylalkohol und Cetylalkohol werden als Bestandteile unter dem Namen Prolipid® 141 vertrieben. Prolipid 141 enthält 24-30% Glycerylstearate, 24-30% Behenylalkohol, 13-19% Palmitinsäure, 11-17% Stearinsäure, 1-7% Lecithin, und je 9-15% Laurylalkohol, Myristylalkohol und Cetylalkohol. Es lässt extrem ölige Formulierungen sich seidig und trocken anfühlen, gibt O/W-Formulierungen eine bessere Struktur und stärkt die Hautbarriere. Der optimale pH-Wert liegt im Bereich von pH 3,8 bis 8,0.

Phenoxyethanol und Ethylhexylglycerin sind Bestandteile des Konservierungsmittelsystems Euxyl ®PE 9010 und als solches erhältlich.

Die Zusammensetzung kann in Form einer Creme, Lotion, Emulsion, insbesondere einer Öl-in-Wasser-Emulsion oder Wasser-in-ÖI-Emulsion, als Mikrokapseln, verwendet werden. Als besonders vorteilhaft hat sich die Anwendung der Zusammensetzung in Form einer mittelviskosen und schnell einziehenden Creme zur lokalen Anwendung erwiesen.

Die vorliegende Zusammensetzung kann als solche oder in Form eines Medikamentes insbesondere Prophylaxe und Behandlung von trockener empfindlicher Haut oder ekzematöser Haut verwendet werden. So führt die Zusammensetzung nach Auftragen auf die Haut zur Verbesserung des äußeren Erscheinungsbildes der Haut. Zudem werden Rötungen und Juckreiz der betroffenen Hautregionen stark reduziert, was zu einer Verbesserung des allgemeinen Wohlbefindens des Anwenders führt. Demnach ist die Zusammensetzung in einem Verfahren zur Behandlung von Neurodermitis geeignet. Durchgeführte Studien zeigen eine signifikante Verbesserung des Hautzustandes der betroffenen Personen.

Die vorliegende Zusammensetzung wird in einem Verfahren mit den folgenden Verfahrensschritten hergestellt:
- Herstellen einer mindestens einen wasserlöslichen Feuchtigkeitsspender enthaltenen wässrigen Phase (Teil A),
- Herstellen einer öligen Phase umfassend mindestens einen Emulgator, mindestens einen Konsistenzgeber, mindestens ein Verdickungsmittel, mindestens ein Konservierungsmittel und/oder mindesten einen Feuchtigkeitsspender (Teil B),
- Vereinigen der wässrigen Phase (Teil A) und der öligen Phase (Teil B) zu einer Emulsion, insbesondere o/w-Emulsion (Teil C),
- Zugabe von Cyanocobalamin, einem ethanolisch-wässrigen *Mahonia aquifolium-*Extrakt und Aloe Vera Gel zur Emulsion und Homogenisieren der Zusammensetzung, bevorzugt unter Vakuum.

Die einzelnen Phasen bzw. Teile der Zusammensetzung werden einzeln und zusammen homogenisiert.

Nach Zugabe der aktiven Komponenten Cyanocobalamin, ethanolisch-wässriger *Mahonia aquifolium-*Extrakt und Aloe Vera Gel zur Emulsion erfolgt bevorzugt die Neutralisation der Emulsion, insbesondere unter Verwendung einer Natriumhydroxidlösung.

Ein wichtiger Faktor im Herstellungsprozess ist die Einhaltung der Reihenfolge der Zugabe der Inhaltsstoffe.

Die vorliegende Erfindung wird im Folgenden eingehender anhand mehrerer Ausführungsbeispiele näher erläutert.

### Ausführungsbeispiel 1

Eine Zusammensetzung mit den folgenden Inhaltsstoffen wurde hergestellt (Chargengröße 100 kg):

| **Nr.** | **Inhaltsstoff** | **Menge [kg]** |
|---|---|---|
| Teil A | Demin. Wasser | 45,55 |
| 1 | | |
| 2 | Glycerin 86,5% | 3,0 |
| 3 | Pemulen TR-2: Acrylates/C10-30 Alkyl Acrylate Crosspolymer | 0,2 |
| Teil B | Sympatens O/2500: Sorbitan Stearate (50-75%), Methyl Glucose Sesquistearate (25-50%) | 4,0 |
| 4 | | |
| 5 | Cutina GMS: Glyceryl Stearate | 1,5 |
| 6 | Prolipid 141 | 3,0 |
| 7 | Nachtkerzenöl raffiniert Ph.Eur. | 30,0 |
| 8 | Lanette O: Cetearyl Alcohol | 2,0 |
| 9 | Lipocire A Pellets: Hydrogenated Palm Kernel Glycerides (>50%), Hydrogenated Palm Glycerides (5-10%) | 2,0 |
| 10 | Keltrol CG-SFT: Xanthan Gum | 0,25 |
| Teil C | Aloe vera Gel 1:10 | 2,0 |
| 11 | | |
| 12 | Mahonia Extrakt std. | 5,0 |
| 13 | Euxyl PE 9010 | 1,1 |
| 14 | Vitamin B12 | 0,1 |
| 15 | Natronlauge 10% | 0,3 |

Zur Herstellung des Teils A (Wasserphase) der obigen Zusammensetzung werden zunächst die Komponenten 1 und 2 (Wasser und Glycerin) in einem Wasserkessel vorgelegt. Anschließend wird Pemulen TR-2 (Komponente 3) langsam eingestreut und ca. 1 h bis zur vollständigen Auflösung gerührt.

Zur Herstellung des Teils B (Ölphase) werden die Komponenten 4 bis 10 im Mischer eingewogen und auf eine Endtemperatur von 75 bis 80°C erhitzt. Anschließend wird Teil A zu Teil B in den Mischer unter Homogenisieren eingezogen und für 5 Minuten auf Stufe II homogenisiert. Es wird 10 min bei Temperatur gerührt, anschließend mit der Kühlung begonnen. Bei ca. 60 °C wird für 5 Minuten auf Stufe II homogenisiert.

Zur Herstellung des Teils C (vereinigte o/w-Emulsion) werden die Komponenten 11-13 zusammen eingewogen, verrührt und unterhalb 40°C zur vereinigten Emulsion gegeben. Anschließend wird das eingewogene Vitamin B12 sehr vorsichtig und langsam zugegeben. Die Natriumhydroxidlösung wird zur Neutralisation langsam zur Emulsion gegeben. Es wird noch einmal für 3 Minuten auf Stufe II homogenisiert und anschließend bis ca. 25°C langsam unter Vakuum kaltgerührt.

Der pH-Wert der Zusammensetzung beträgt 5,2, die Viskositätswerte liegen bei ca. 300.000 mpas (Sp93/2,5, (Brookfield), die Dichte beträgt ca. 0,8696 g/cm3.

Die vorliegende Rezeptur wurde auf Stabilität und Lagerfähigkeit geprüft mit in der Kosmetik bekannten Methoden (thermischer Stresstest, Schaukeltest zwischen -5 und + 40°C, UV-Einwirkung, Lagerung bei Raumtemperatur und bei 40°C). Das Produkt entspricht allen Anforderungen incl. mikrobiologischer Stabilität.

### Ausführungsbeispiel 2

Die Wirksamkeit der Zusammensetzung wurde an 20 Testpersonen, die neurodermitische Beschwerden in Form von atopischen, leicht entzündlichen Stellen aufweisen, über einen Testzeitraum von 2 und 4 Wochen untersucht.

### Epikutantest

Alle 20 Testpersonen vertrugen das Produkt einwandfrei. Es kam in keinem Fall zu unerwünschten oder gar pathologischen Hautveränderungen. Zusammenfassend kann gesagt werden, dass das Produkt im vierwöchigen Anwendungstest unter klinischdermatologischen Bedingungen sehr gut vertragen wurde.

### Optische 3 D Messung der Hautoberfläche

Nach 2 Wochen verringerte sich die Faltentiefe nach Anwendung der Zusammensetzung um durchschnittlich 31,84 % (Minimum: -19,64 % Maximum: -47,32 %), d.h. es trat eine Verbesserung bei allen Probanden ein. Nach 4 Wochen war ein Verringerung der Faltentiefe nach Anwendung des Präparats um durchschnittlich 35,20 % nachweisbar (Minimum: 15,64 % Maximum: -47,32 %)

Somit bewirkt die Zusammensetzung bereits nach 2 Wochen eine Verbesserung der Beschaffenheit der Hautoberfläche bei allen Probanden. In der Langzeitanwendung war eine weitere kleine Verbesserung des Ergebnisses nachweisbar.

### Atopic Score modifiziert nach SCORAD

Es erfolgte die Messung eines Index zur Bestimmung des Schweregrades des atopischen Ekzems (SCORAD= Severety Scoring of Atopic Dermatitis). Der Wert des Atopic Score setzt sich zusammen aus dermatologischen Beurteilungskriterien (typische morphologische Veränderungen, Anteil der betroffenen Hautfläche bzgl Rötung, Kruste, Excoration) und subjektiven Einschätzungen des Probanden (Einschätzung Juckreiz und Schlafverlust).

Bei Anwendung der Zusammensetzung ergab sich nach 2 Wochen eine Verringerung des Atopic Scores um 36,86 % und nach 4 Wochen eine Verringerung des Atopic Scores um 81,83 %.

Nach 4 Wochen war der Score bei 11 Probanden durch die Behandlung auf einen Wert von 0 zurückgegangen ist, d.h. es treten weder subjektive noch objektive Krankheitssymptome nach der 4wöchigen Behandlung bei über der Hälfte der Probanden auf (zu Beginn der Studie hatten alle Probanden leichte Hautveränderungen der Neurodermitis).

### Hautfettbestimmung mittels Sebumeter

Nach einen Zeitraum von 2 Wochen Anwendung der Zusammensetzung stieg der Hautfettgehalt um 25,64 % und nach 4 Wochen um 40,08 %.

Insgesamt wurden die Hautfetteigenschaften bereits nach 2 Wochen verbessert. Eine Anwendung über einen längeren Zeitraum verbesserte die Hautfetteigenschaften noch mal fast um das doppelte.

### Hvdrationsbestimmuna mit dem Corneometer

Die Messung der Hautfeuchtigkeit der äußeren Schicht der Oberhaut ergab nach Anwendung der Zusammensetzung über einen Zeitraum von 2 Wochen eine Verbesserung der Hautfeuchtigkeit um 27,65 % und nach 4 Wochen eine Verbesserung der Hautfeuchtigkeit um 38,57 %.

Somit wurden die Hautfeuchtigkeitseigenschaften wurden bereits nach 2 Wochen verbessert. Auch nach Anwendung über einen längeren Zeitraum wurden die Feuchtigkeitseigenschaften der Haut durchschnittlich noch mal verbessert wurden.

### Transepidermaler Wasserverlust (TEWL)

Der transepidermale Wasserverlust der Haut ist einer der wichtigsten Parameter zur Beurteilung der Schutzfunktion der menschlichen Haut. Dabei handelt es sich um die Abdunstung von Wasser aus dem Körperinneren ohne Einbeziehung des Wasserverlustes durch Schwitzen. Ein Präparat, das die Hautbarrierefunktion aufrecht erhält bzw. verbessert, bewirkt eine Reduzierung des TEWL. Eine geringere Wasserverdunstung auf der Haut entspricht einer guten Feuchthaltewirkung der Haut.

Die Anwendung der Zusammensetzung über einen Zeitraum von 2 Wochen führte zu einer Verminderung des TEWL um 6,08 % und nach 4 Wochen um 10,84 %, so dass auch hier von einer Verbesserung der Feuchthaltewirkung der Haut ausgegangen werden kann.

Zusammenfassend ist zu sagen, dass die Studien eine signifikante Verbesserung des Hautzustandes der an den Studien beteiligten Atopicer aufzeigen.

## Patentansprüche

1. Kosmetische Zusammensetzung **umfassend**
- 0,01 bis 2 Gew% Cyanocobalamin,
- 0,01 bis 5 Gew% von mindestens einem ethanolisch-wässrigen Extrakt standardisiert auf die Leitsubstanz Berberin aus der Pflanze *Mahonia aquifolium* , wobei der Anteil an Berberin im Extrakt mehr als 0,5 mg/ml beträgt,
- 1 bis 50 Gew% eines Aloe vera Gels, sowie
- Nachtkerzenöl, Acrylate / C10-C30 Alkylacrylatcrosspolymer und Glyceride.

2. Zusammensetzung nach einem der vorhergehenden Ansprüche, **gekennzeichnet durch** mindestens einen Emulgator, mindestens einen Konsistenzgeber, mindestens ein Verdickungsmittel und/oder mindestens ein Konservierungsmittel.

3. Zusammensetzung nach einem der vorhergehenden Ansprüche, **gekennzeichnet durch** 0,05 bis 1,5 Gew %, insbesondere bevorzugt 0,1 bis 1 Gew% Cyanocobalamin.

4. Zusammensetzung nach einem der vorhergehenden Ansprüche, **gekennzeichnet durch** 0,05 bis 2 Gew%, insbesondere bevorzugt 0,1 bis 1 Gew% eines ethanolisch-wässrigen Extraktes aus der Pflanze *Mahonia aquifolium.*

5. Zusammensetzung nach einem der vorhergehenden Ansprüche, **gekennzeichnet durch** 5 bis 30 Gew%, insbesondere bevorzugt 10 bis 25 Gew% Aloe vera Gel.

6. Zusammensetzung nach einem der vorhergehenden Ansprüche, **dadurch gekennzeichnet, dass** die Zusammensetzung in Form einer Creme, Lotion oder Emulsion, insbesondere einer Öl-in-Wasser-Emulsion oder Wasser-in-ÖI-Emulsion, verwendet wird.

7. Zusammensetzung nach einem der vorhergehenden Ansprüche zur Verwendung bei der Prophylaxe und Behandlung von trockener empfindlicher Haut oder ekzematöser Haut.

8. Verfahren zur Herstellung einer kosmetischen Zusammensetzung nach einem der Ansprüche 1 bis 7 umfassend die Schritte:
- Herstellen einer mindestens einen wasserlöslichen Feuchtigkeitsspender enthaltenen wässrigen Phase (Teil A),
- Herstellen einer öligen Phase umfassend mindestens einen Emulgator, mindestens einen Konsistenzgeber, mindestens ein Verdickungsmittel, mindestens ein Konservierungsmittel und/oder mindesten ein Feuchtigkeitsspender (Teil B),
- Vereinigen der wässrigen Phase (Teil A) und der öligen Phase (Teil B) zu einer Emulsion, insbesondere o/w-Emulsion (Teil C),
- Zugabe von Cyanocobalamin, einem ethanolisch-wässrigen Mahonia aquifolium-Extrakt und Aloe Vera Gel zur Emulsion und Homogenisieren der Zusammensetzung, bevorzugt unter Vakuum.

9. Verfahren nach Anspruch 8, **dadurch gekennzeichnet, dass** nach Zugabe des Cyanocobalamins und des ethanolisch-wässrigen *Mahonia aquifolium*-Extraktes die Zusammensetzung durch Zugabe von mindestens einer alkalischen Verbindung neutralisiert wird.

## Claims

1. A cosmetic composition, **comprising**
- 0.01 to 2 wt-% cyanocobalamin,
- 0.01 to 5 wt-% of at least one ethanolic aqueous extract standardized to the lead substance berberine from the plant *Mahonia aquifolium,* wherein the amount of berberine in the extract is more than 0.5 mg/ml,
- 1 to 50 wt-% of an aloe vera gel, and
- evening primrose oil, acrylate / C₁₀-C₃₀ alkyl acrylate cross-polymer and glycerides.

2. The composition according to any of the preceding claims, **characterized by** at least one emulsifier, at least one consistency enhancer, at least one thickening agent and/or at least one preservative.

3. The composition according to any of the preceding claims, **characterized by** 0.05 to 1.5 wt.%, in particular preferably 0.1 to 1 wt-% cyanocobalamin.

4. The composition according to any of the preceding claims, **characterized by** 0.05 to 2 wt-%, in particular preferably 0.1 to 1 wt-% of an ethanolic aqueous extract from the plant *Mahonia aquifolium.*

5. The composition according to any of the preceding claims, **characterized by** 5 to 30 wt-%, in particular preferably 10 to 25 wt-% aloe vera gel.

6. The composition according to any of the preceding claims, **characterized in that** the composition is used in the form of a cream, lotion or emulsion, in particular an oil-in-water emulsion or water-in-oil emulsion.

7. The composition according to any of the preceding claims for use in the prophylaxis and treatment of dry sensitive skin or eczematous skin.

8. A method for the preparation of a cosmetic composition according to any of claims 1 to 7, comprising the steps:
- preparation of an aqueous phase containing at least one water-soluble moisturizer (part A),
- preparation of an oily phase comprising at least one emulsifier, at least one consistency enhancer, at least one thickening agent, at least one preservative and/or at least one moisturizer (part B),
- combining the aqueous phase (part A) and the oily phase (part B) to obtain an emulsion, in particular an oil-in-water emulsion (part C),
- addition of cyanocobalamin, an ethanolic aqueous *Mahonia aquifolium* extract and aloe vera gel to the emulsion and homogenizing the composition, preferably under vacuum.

9. The method according to claim 8, **characterized in that** after the addition of the cyanocobalamin and the ethanolic aqueous *Mahonia aquifolium* extract the composition is neutralized by the addition of at least one alkaline compound.

## Revendications

1. Composition cosmétique comprenant
- 0,01 à 2 % en poids de cyanocobalamine,
- 0,01 à 5 % en poids d'au moins un extrait éthanolique-aqueux normalisé par rapport au principe actif berbérine issu de la plante *Mahonia aquifolium,* dans laquelle la proportion de berbérine dans l'extrait est de plus de 0,5 mg/ml,
- 1 à 50 % en poids d'un gel d'aloe vera, ainsi que
- de l'huile d'onagre, un polymère réticulé acrylates / C10-C30 alkylacrylate et des glycérides.

2. Composition selon l'une des revendications précédentes, **caractérisée par** au moins un émulsifiant, au moins un agent de consistance, au moins un épaississant et/ou au moins un conservateur.

3. Composition selon l'une des revendications précédentes, **caractérisée par** 0,05 à 1,5 % en poids, en particulier de préférence de 0,1 à 1 % en poids de cyanocobalamine.

4. Composition selon l'une des revendications précédentes, **caractérisée par** 0,05 à 2 % en poids, en particulier de préférence de 0,1 à 1 % en poids d'un extrait éthanolique-aqueux issu de la plante *Mahonia aquifolium.*

5. Composition selon l'une des revendications précédentes, **caractérisée par** 5 à 30 % en poids, en particulier de préférence 10 à 25 % en poids de gel d'aloe vera.

6. Composition selon l'une des revendications précédentes, **caractérisée en ce que** la composition est utilisée sous la forme d'une crème, d'une lotion ou d'une émulsion, en particulier d'une émulsion huile dans eau ou d'une émulsion eau dans huile.

7. Composition selon l'une des revendications précédentes, pour son utilisation dans la prophylaxie et le traitement de la peau sèche et sensible ou de la peau exémateuse.

8. Procédé de fabrication d'une composition cosmétique selon l'une des revendications 1 à 7 comprenant les étapes de :
- fabrication d'une phase aqueuse contenant au moins un agent hydratant hydrosoluble (partie A),
- fabrication d'une phase huileuse comprenant au moins un émulsifiant, au moins un agent de consistance, au moins un épaississant, au moins un conservateur et/ou au moins un agent hydratant (partie B),
- réunion de la phase aqueuse (partie A) et de la phase huileuse (partie B) en une émulsion, en particulier, une émulsion h/e (partie C),
- addition de cyanocobalamine, d'un extrait éthanolique-aqueux de *Mahonia aquifolium* et de gel d'aloe vera à l'émulsion et l'homogénéisation de la composition, de préférence sous vide.

9. Procédé selon la revendication 8, **caractérisé en ce qu'**après l'addition de la cyanocobalamine et de l'extrait éthanolique-aqueux de *Mahonia aquifolium,* la composition est neutralisée par l'addition d'au moins un composé alcalin.
